# EUROPEAN PATENT SPECIFICATION

(11) **EP 3 866 722 B1**
(45) Date of publication and mention of the grant of the patent: **11.06.2025**
(21) Application number: 19873427.9
(22) Date of filing: 11.10.2019
(51) Int. Cl.: A61B 50/20, A61B 50/24, A61M 5/00, A61B 50/22, A61B 50/30

(54) **STERILE STAND FOR SUPPORTING SURGICAL INSTRUMENTS**
STERILER STÄNDER ZUM TRAGEN VON CHIRURGISCHEN INSTRUMENTEN
SUPPORT STÉRILE POUR SUPPORTER DES INSTRUMENTS CHIRURGICAUX

(30) Priority: 16.10.2018 US 201816161411
(43) Date of publication of application: 25.08.2021
(73) Proprietor: Misle, Gayle, Millbrae, CA 94030 (US)
(72) Inventor: Misle, Gayle, Millbrae, CA 94030 (US)
(74) Representative: Hancox, Jonathan Christopher
(86) International application number: PCT/US2019/055869
(87) International publication number: WO 2020/081390

(56) References cited:
- EP-A1- 3 042 626
- WO-A1-2014/162272
- US-A- 2 666 967
- US-A- 2 741 048
- US-A- 2 810 473
- US-A- 5 823 363
- US-A- 5 833 057
- US-A- 5 850 917
- US-A1- 2017 303 956

## Description

### FIELD OF THE INVENTION

This invention relates to a sterile stand for supporting surgical instruments or medical tools such as epidural needles, blunt-tip cannulas, syringes and injection needle assemblies.

### DESCRIPTION OF THE RELATED ART

Applicants have previously filed U.S. Patent Application Serial No.15/133,660 filed April 20, 2016 entitled CANNULA AND NEEDLE ASSEMBLY and U.S. Patent Application Serial No. 15/354,249 filed November 17, 2016 entitled EPIDURAL NEEDLE ASSEMBLY. In U. S. Patent Application Serial No. 15/354,249, during the use of components thereof, there are occasions when the epidural needle or cannula needs to be removed from the patient to add additional local anesthetic or fillers to a syringe connected to the epidural needle or cannula. It is important during this procedure to maintain the epidural needle or cannula in a sterile condition if it is to be used again. Prior to the invention of the co-pending application, maintaining the needle in a sterile condition required a two-handed technique. In the prior art procedure, one hand holds the syringe with the other hand removes the needle cap from the syringe. Then, the person must put the syringe down with one hand while the other hand grasps the needle cap and places it on the needle to keep it sterile. The invention of the co-pending application represents an improvement in the art. The instant invention represents a further improvement in the art.

The following documents relate to devices in the general field of the invention. US 2,666,967 (Poitras) discloses a lancet readying and storing device. US 5,850,917 (Denton et al.) discloses a syringe dosage tracking device with cooling feature. US 5,823,363 discloses a medical syringe holding/transport apparatus. US 2,810,473 discloses a sterile hypodermic needle holder. WO 2014/162272 discloses a supporting base for medial instruments. EP 3,042,626 discloses a medical device tray.

### SUMMARY OF THE INVENTION

The present invention provides a sterile stand for supporting sterile surgical instruments in a vertically-disposed sterile manner in accordance with claim 1.

A sterile stand for supporting surgical instruments in a vertically disposed sterile manner is disclosed. The stand includes a solid housing having a horizontally disposed lower end, a horizontally disposed upper end, and upstanding sides extending between the lower and upper ends of the housing. First, second, third, fourth, fifth and sixth openings are formed in the upper end of the housing. An elongated and vertically disposed first cylindrical bore is formed in the housing and which has upper and lower ends. The upper end of the first cylindrical bore is in communication with the first opening and extends downwardly therefrom towards the bottom of the housing whereby the lower end of the first cylindrical bore is closed. An elongated and vertically disposed second cylindrical bore is formed in the housing which has upper and lower ends. The upper end of the second cylindrical bore is in communication with the second opening and extends downwardly therefrom towards the bottom of the housing whereby the lower end of the second cylindrical bore is closed. The first opening in the upper end of the housing and the first cylindrical bore are configured to have a first syringe selectively positioned therein. The second opening in the upper end of the housing and the second cylindrical bore are configured to have a second syringe selectively positioned therein.

The housing also includes an elongated and vertically disposed third cylindrical bore having upper and lower ends. The upper end of the third cylindrical bore is in communication with the third opening and extends downwardly therefrom towards the bottom of the housing whereby the lower end of the third cylindrical bore is closed. The third opening and the third cylindrical bore are configured to have a blunt-tip cannula or epidural needle positioned therein. The housing also includes an elongated and vertically disposed fourth cylindrical bore having upper and lower ends. The upper end of the fourth cylindrical bore is in communication with the fourth opening and extends downwardly therefrom towards the bottom of the housing whereby the lower end of the fourth cylindrical bore is closed. The fourth opening and the fourth cylindrical bore are configured to have a blunt-tip cannula or epidural needle positioned therein. The housing also includes an elongated fifth cylindrical bore having upper and lower ends. The upper end of the fifth cylindrical bore is in communication with the fifth opening and extends downwardly therefrom towards the bottom of the housing whereby the lower end of the fifth cylindrical bore is closed. A horizontally disposed first slot is formed in the upper end of the housing which has an upper end and a lower end with the first slot extending laterally from the fifth opening in the upper end of the housing. The fifth opening and the first slot are configured to have an injection needle assembly selectively positioned therein. The housing also includes an elongated sixth cylindrical bore having upper and lower ends. The upper end of the sixth cylindrical bore is in communication with the sixth opening and extends downwardly towards the bottom of the housing whereby the lower end of the side to sixth bore is closed. A horizontally disposed second slot is formed in the upper end of the housing which has an upper end and a lower end with the second slot extending laterally from the sixth opening. The sixth opening and the second slot are configured to have an injection needle assembly selectively positioned therein.

In one embodiment of the invention, the stand is square-shaped. In another embodiment of the invention, the stand is cylindrical-shaped. In yet another embodiment of the invention, the side walls of the stand are curved.

It is therefore a principal object of the invention to provide an improved sterile stand for supporting surgical instruments or medical tools therein.

A further object of the invention is to provide a sterile stand for supporting epidural needles, cannulas, and syringes therein and for supporting an injection needle assembly therein.

These and other objects will be apparent to those skilled in the art.

### BRIEF DESCRIPTION OF THE DRAWINGS

Non-limiting and non-exhaustive embodiments of the present invention are described with reference to the following figures, wherein like reference numerals refer to like parts throughout the various views unless otherwise specified.

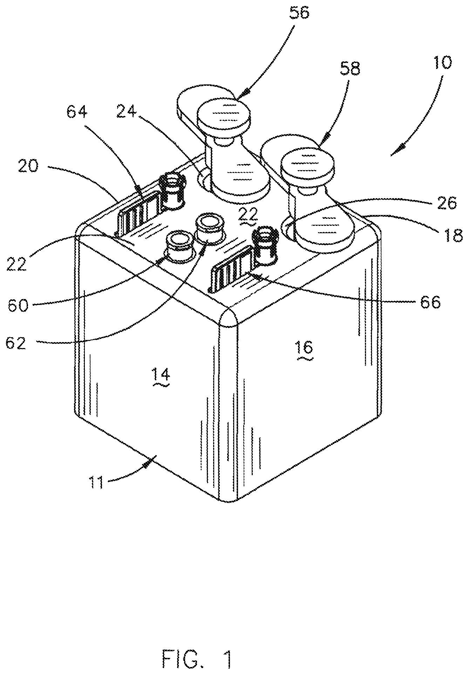

Fig. 1 is a perspective view of one embodiment of the stand of this invention and which illustrates various surgical instruments or medical tools positioned therein;

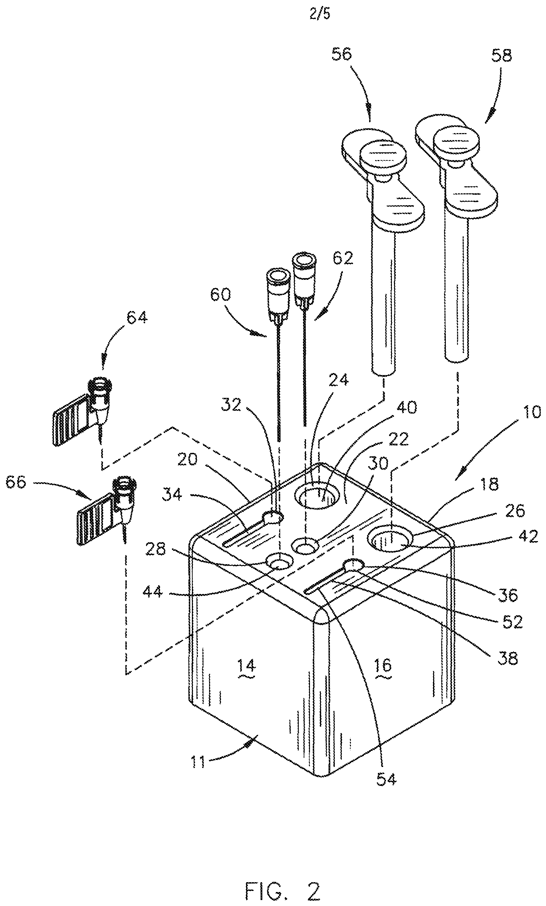

Fig. 2 is an exploded perspective view of the embodiment of Fig.1;

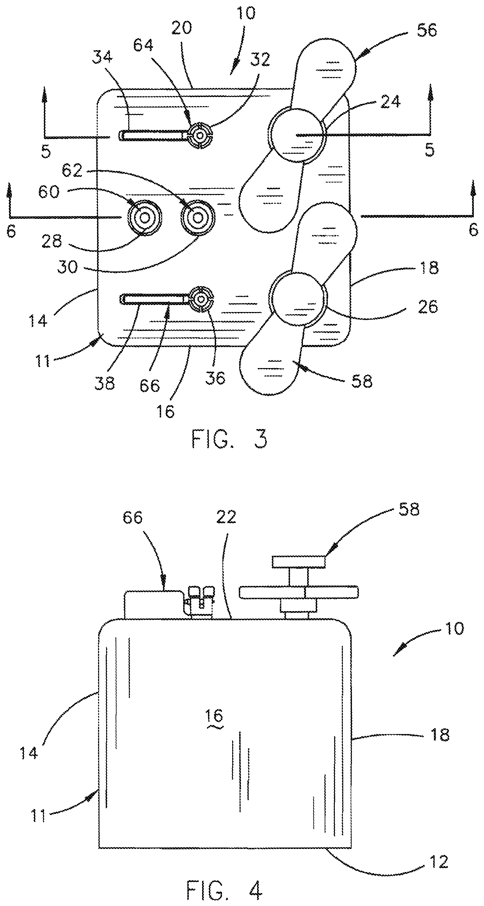

Fig. 3 is a top view of the stand of this invention;
Fig. 4 is a side view of the stand of this invention;

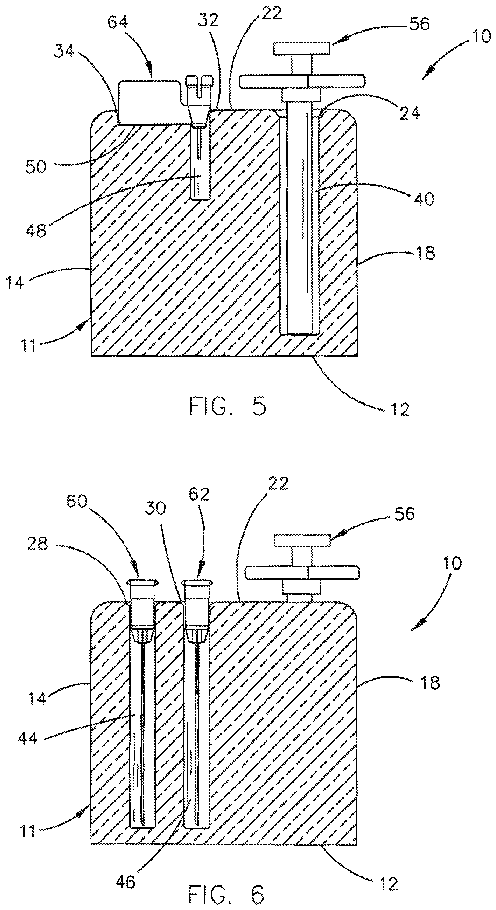

Fig. 5 is a sectional view as seen along lines 5-5 of Fig. 3;
Fig. 6 is a sectional view as seen along lines 6-6 of Fig. 3;
Fig. 7 is a perspective view of a modified form of the stand; and
Fig. 8 is a modified version of the stand of this invention.

### DESCRIPTION OF THE PREFERRED EMBODIMENT

Embodiments are described more fully below with reference to the accompanying figures, which form a part hereof and show, by way of illustration, specific exemplary embodiments. These embodiments are disclosed in sufficient detail to enable those skilled in the art to practice the invention. However, embodiments may be implemented in many different forms and should not be construed as being limited to the embodiments set forth herein. The following detailed description is, therefore, not to be taken in a limiting sense in that the scope of the present invention is defined only by the appended claims.

The numeral 10 refers to the sterile support of this invention which includes a housing 11. Although the support 10 is preferably square-shaped as seen in the drawings, support 10 could have a rectangular shape, a cylindrical shape, or some other shape as will be pointed out hereinafter. Support 10 is of solid block configuration and is comprised of a suitable plastic material. Housing 11 includes a horizontally disposed lower end 12 and vertically disposed sides 14, 16, 18 and 20. Housing 11 also includes an upper end 22 which extends between the upper ends of sides 14, 16, 18 and 20.

Upper end 22 has a pair of spaced-apart chamfered openings 24 and 26 formed therein. Upper end 22 also has a pair of spaced-apart chamfered openings 28 and 30 formed therein. Upper end 22 also has a chamfered opening 32 formed therein. An elongated and horizontally disposed slot 34 is formed in the upper end 22 of housing 11 which extends laterally from opening 32. Upper end 22 also has a chamfered opening 36 formed therein. An elongated and horizontally disposed slot 38 is formed in upper end 22 of housing 11 which extends laterally from opening 36.

The numeral 40 refers to an elongated cylindrical bore in housing 11 which extends downwardly from the chamfered opening 24 towards the bottom 12 of the housing 11 whereby the lower end of bore 40 is closed. The numeral 42 refers to an elongated cylindrical bore formed in housing 11 which extends downwardly from the chamfered opening 26 towards the bottom 12 of the housing 11 whereby the lower end of bore 42 is closed. The numeral 44 refers to an elongated cylindrical bore formed in housing 11 which extends downwardly from the chamfered opening 28 towards the bottom 12 of housing 11 whereby the lower end of bore 44 is closed. The numeral 46 refers to an elongated cylindrical bore formed in housing 11 which extends downwardly from the chamfered opening 30 towards the bottom 12 of housing 11 whereby the lower end of bore 46 is closed.

An elongated cylindrical bore 48 is formed in housing 11 which extends downwardly from opening 32. An elongated slot 50 is formed in housing 11 which extends downwardly from slot 34. An elongated cylindrical bore 52 is formed in housing 11 which extends downwardly from opening 36. An elongated slot 54 is formed in housing 11 which extends downwardly from slot 38.

The numeral 56 refers to a syringe which may be selectively positioned in opening 24 and bore 40. The numeral 58 refers to a syringe which may be selectively positioned in opening 26 and bore 42. The numeral 60 refers to a blunt-tip cannula or epidural needle which may be positioned in opening 28 and bore 44. The numeral 62 refers to a blunt-tip cannula or epidural needle which may be positioned in opening 30 and bore 46. The numeral 64 refers to an injection needle assembly which may be positioned in opening 32, bore 48, slot 34 and slot 50. The numeral 66 refers to an injection needle assembly which may be positioned in opening 36, bore 52, slot 38 and slot 54.

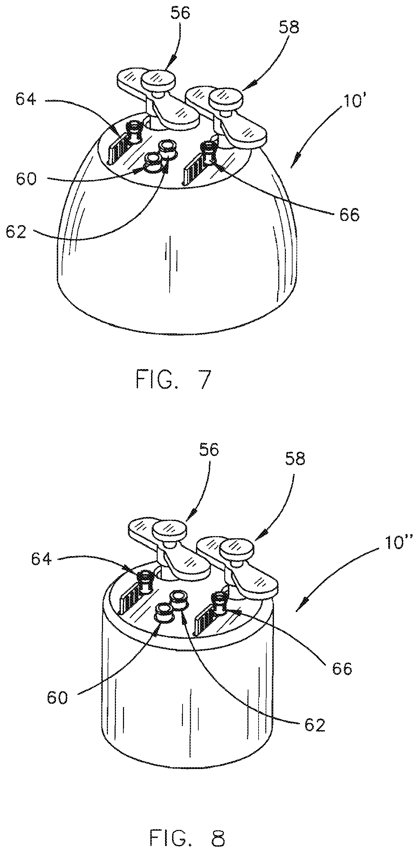

As stated above, the stand 10 may have other shapes. For example, see Fig. 7 wherein the stand 10' has curved sides. Fig. 8 illustrates another embodiment of the stand 10' wherein the sides of the stand are cylindrical.

Thus it can be seen that an improved stand has been provided for supporting surgical instruments or medical tools therein.

Thus it can be seen that the invention accomplishes at least all of its stated objectives.

## Claims

1. A sterile stand (10, 10', 10") for supporting sterile surgical instruments in a vertically disposed sterile manner, comprising:
a sterile housing (11) including a horizontally disposed upper end (22) and a lower end (12);
a first opening (24) formed in said upper end (22) of said sterile housing (11);
a second opening (26) formed in said upper end (22) of said sterile housing (11);
a third opening (28) formed in said upper end (22) of said sterile housing (11);
a fourth opening (30) formed in said upper end (22) of said sterile housing (11);
a fifth opening (32) formed in said upper end (22) of said sterile housing (11);
a first elongated and horizontally disposed slot (34) formed in said upper end (22) of said sterile housing (11), which extends laterally from said fifth opening (32);
a sixth opening (36) formed in said upper end (22) of said sterile housing (11);
a second elongated and horizontally disposed slot (38) formed in said upper end (22) of said sterile housing (11) which extends laterally from said sixth opening (36);
an elongated and vertically disposed first cylindrical bore (40) formed in said sterile housing (11) which has upper and lower ends;
said upper end of said first cylindrical bore (40) being in communication with said first opening (24) and extending downwardly therefrom towards said bottom of said sterile housing (11) whereby said lower end of said first cylindrical bore (40) is closed;
an elongated and vertically disposed second cylindrical bore (42) formed in said sterile housing (11) which has upper and lower ends;
said upper end of said second cylindrical bore (42) being in communication with said second opening (26) and extending downwardly therefrom towards said bottom of said sterile housing (11) whereby said lower end of said second cylindrical bore (42) is closed;
said first opening (24) in said upper end (22) of said sterile housing (11) and said first cylindrical bore (40) being configured to have an elongated first sterile syringe (56), with upper and lower ends, selectively positioned therein;
said second opening (26) in said upper end (22) of said sterile housing (11) and said second cylindrical bore (42) being configured to have an elongated second sterile syringe (58), with upper and lower ends, selectively positioned therein;
an elongated and vertically disposed third cylindrical bore (44) having upper and lower ends;
said upper end of said third cylindrical bore (44) being in communication with said third opening (28) and extending downwardly therefrom towards said bottom of said sterile housing (11) whereby said lower end of said third cylindrical bore (44) is closed;
said third opening (28) and said third cylindrical bore (44) being configured to have an elongated and sterile blunt-tip cannula or a sterile epidural needle (60), having upper and lower ends, positioned therein;
an elongated and vertically disposed fourth cylindrical bore (46) having upper and lower ends;
said upper end of said fourth cylindrical bore (46) being in communication with said fourth opening (30) and extending downwardly therefrom towards said bottom of said sterile housing (11) whereby said lower end of said fourth cylindrical bore (46) is closed;
said fourth opening (30) and said fourth cylindrical bore (46) being configured to have an elongated and sterile blunt-tip cannula or a sterile epidural needle (62), having upper and lower ends, positioned therein;
an elongated fifth cylindrical bore (48) having upper and lower ends;
said upper end of said fifth cylindrical bore (48) being in communication with said fifth opening (32) and extending downwardly therefrom towards said bottom of said sterile housing (11) whereby said lower end of said fifth cylindrical bore (48) is closed;
said upper end (22) of said sterile housing (11) being spaced above said lower end of said sterile housing (11) whereby said upper ends of said first and second elongated and sterile syringes (56, 58) are positioned adjacent said upper end (22) of said sterile housing (11) when said first and second elongated and sterile syringes (56, 58) are positioned in said first opening (24) and said first cylindrical bore (40) and said second opening (26) and said second cylindrical bore (42) respectively;
said upper end (22) of said sterile housing (11) being spaced above said lower end of said sterile housing (11) whereby said upper ends of said elongated and sterile blunt-tip cannulas or sterile epidural needles (60, 62) are positioned adjacent said upper end (22) of said sterile housing (11) when said sterile blunt-tip cannulas or sterile epidural needles (60, 62) are positioned in said third opening (28) and said third bore (44) and said fourth opening (30) and said fourth bore (46) respectively;
said horizontally disposed first slot (34) in said sterile housing (11) having an upper end and a lower end;
said fifth opening (32), said fifth cylindrical bore (48) and said first slot (34) being configured to have a sterile injection needle assembly (64), with upper and lower ends, selectively positioned therein;
an elongated sixth cylindrical bore (52) having upper and lower ends;
said upper end of said sixth cylindrical bore (52) being in communication with said sixth opening (36);
said horizontally disposed second slot (38) in said sterile housing (11) having an upper end and a lower end;
said sixth opening (36), said sixth cylindrical bore (52) and said second slot (38) being configured to have a sterile injection needle assembly (66) selectively positioned therein;
the upper end of the sterile injection needle assembly (64) being positioned at the upper end (22) of said sterile housing (11) when the sterile injection needle assembly (64) is positioned in said fifth opening (32), said fifth cylindrical bore (48) and said first slot (34); and
the upper end of the sterile injection needle assembly (66) being positioned at the upper end (22) of said sterile housing (11) when a sterile injection needle assembly (66) is positioned in said sixth opening (36), said sixth cylindrical bore (52) and said second slot (38).

## Patentansprüche

1. Steriler Ständer (10, 10', 10") zum Tragen steriler chirurgischer Instrumente in einer vertikal angeordneten sterilen Weise, umfassend:
ein steriles Gehäuse (11) einschließlich einem horizontal angeordneten oberen Ende (22) und einem unteren Ende (12);
eine erste Öffnung (24), die in dem oberen Ende (22) des sterilen Gehäuses (11) ausgebildet ist;
eine zweite Öffnung (26), die in dem oberen Ende (22) des sterilen Gehäuses (11) ausgebildet ist;
eine dritte Öffnung (28), die in dem oberen Ende (22) des sterilen Gehäuses (11) ausgebildet ist;
eine vierte Öffnung (30), die in dem oberen Ende (22) des sterilen Gehäuses (11) ausgebildet ist;
eine fünfte Öffnung (32), die in dem oberen Ende (22) des sterilen Gehäuses (11) ausgebildet ist;
einen ersten länglichen und horizontal angeordneten Schlitz (34), der in dem oberen Ende (22) des sterilen Gehäuses (11) ausgebildet ist und sich seitlich von der fünften Öffnung (32) erstreckt;
eine sechste Öffnung (36), die in dem oberen Ende (22) des sterilen Gehäuses (11) ausgebildet ist;
einen zweiten länglichen und horizontal angeordneten Schlitz (38), der in dem oberen Ende (22) des sterilen Gehäuses (11) ausgebildet ist und sich seitlich von der sechsten Öffnung (36) erstreckt;
eine längliche und vertikal angeordnete erste zylindrische Bohrung (40), die in dem sterilen Gehäuse (11) ausgebildet ist und ein oberes und ein unteres Ende aufweist;
wobei das obere Ende der ersten zylindrischen Bohrung (40) mit der ersten Öffnung (24) in Verbindung steht und sich von dieser nach unten in Richtung des Bodens des sterilen Gehäuses (11) erstreckt, wodurch das untere Ende der ersten zylindrischen Bohrung (40) verschlossen ist;
eine längliche und vertikal angeordnete zweite zylindrische Bohrung (42), die in dem sterilen Gehäuse (11) ausgebildet ist und ein oberes und ein unteres Ende aufweist;
wobei das obere Ende der zweiten zylindrischen Bohrung (42) mit der zweiten Öffnung (26) in Verbindung steht und sich von dieser nach unten in Richtung des Bodens des sterilen Gehäuses (11) erstreckt, wodurch das untere Ende der zweiten zylindrischen Bohrung (42) verschlossen ist;
wobei die erste Öffnung (24) in dem oberen Ende (22) des sterilen Gehäuses (11) und die erste zylindrische Bohrung (40) so konfiguriert sind, dass sie eine längliche erste sterile Spritze (56) mit einem oberen und einem unteren Ende aufweisen, die gezielt darin positioniert sind;
wobei die zweite Öffnung (26) in dem oberen Ende (22) des sterilen Gehäuses (11) und die zweite zylindrische Bohrung (42) so konfiguriert sind, dass sie eine längliche zweite sterile Spritze (58) mit einem oberen und einem Ende aufweisen, die gezielt darin positioniert sind;
eine längliche und vertikal angeordnete dritte zylindrische Bohrung (44) mit einem oberen und einem unteren Ende;
wobei das obere Ende der dritten zylindrischen Bohrung (44) mit der dritten Öffnung (28) in Verbindung steht und sich von dieser nach unten in Richtung des Bodens des sterilen Gehäuses (11) erstreckt, wodurch das untere Ende der dritten zylindrischen Bohrung (44) verschlossen ist;
wobei die dritte Öffnung (28) und die dritte zylindrische Bohrung (44) so konfiguriert sind, dass sie eine längliche und sterile Kanüle mit stumpfer Spitze oder eine darin positionierte sterile Epiduralnadel (60) mit einem oberen und einem unteren Ende aufweisen;
eine längliche und vertikal angeordnete vierte zylindrische Bohrung (46) mit einem oberen und einem unteren Ende;
wobei das obere Ende der vierten zylindrischen Bohrung (46) mit der vierten Öffnung (30) in Verbindung steht und sich von dieser nach unten in Richtung des Bodens des sterilen Gehäuses (11) erstreckt, wodurch das untere Ende der vierten zylindrischen Bohrung (46) verschlossen ist;
wobei die vierte Öffnung (30) und die vierte zylindrische Bohrung (46) so konfiguriert sind, dass sie eine längliche und sterile Kanüle mit stumpfer Spitze oder eine darin positionierte sterile Epiduralnadel (62) mit einem oberen und einem unteren Ende aufweisen;
eine längliche fünfte zylindrische Bohrung (48) mit einem oberen und einem unteren Ende;
wobei das obere Ende der fünften zylindrischen Bohrung (48) mit der fünften Öffnung (32) in Verbindung steht und sich von dieser nach unten in Richtung des Bodens des sterilen Gehäuses (11) erstreckt, wodurch das untere Ende der fünften zylindrischen Bohrung (48) verschlossen ist;
wobei das obere Ende (22) des sterilen Gehäuses (11) über dem unteren Ende des sterilen Gehäuses (11) beabstandet ist, wodurch die oberen Enden der ersten und der zweiten länglichen und sterilen Spritze (56, 58) benachbart zu dem oberen Ende (22) des sterilen Gehäuses (11) positioniert sind, wenn die erste und die zweite längliche und sterile Spritze (56, 58) in der ersten Öffnung (24) und der ersten zylindrischen Bohrung (40) bzw. in der zweiten Öffnung (26) und der zweiten zylindrischen Bohrung (42) positioniert sind;
wobei das obere Ende (22) des sterilen Gehäuses (11) über dem unteren Ende des sterilen Gehäuses (11) beabstandet ist, wodurch die oberen Enden der länglichen und sterilen Kanülen mit stumpfer Spitze oder sterilen Epiduralnadeln (60, 62) benachbart zu dem oberen Ende (22) des sterilen Gehäuses (11) positioniert sind, wenn die sterilen Kanülen mit stumpfer Spitze oder sterilen Epiduralnadeln (60, 62) in der dritten Öffnung (28) und der dritten Bohrung (44) bzw. in der vierten Öffnung (30) und der vierten Bohrung (46) positioniert sind;
wobei der horizontal angeordnete erste Schlitz (34) in dem sterilen Gehäuse (11) ein oberes Ende und ein unteres Ende aufweist;
wobei die fünfte Öffnung (32), die fünfte zylindrische Bohrung (48) und der erste Schlitz (34) so konfiguriert sind, dass sie eine sterile Injektionsnadelanordnung (64) mit einem oberen und einem Ende aufweisen, die gezielt darin positioniert sind;
eine längliche sechste zylindrische Bohrung (52) mit einem oberen und einem unteren Ende;
wobei das obere Ende der sechsten zylindrischen Bohrung (52) mit der sechsten Öffnung (36) in Verbindung steht;
wobei der horizontal angeordnete zweite Schlitz (38) in dem sterilen Gehäuse (11) ein oberes Ende und ein unteres Ende aufweist;
wobei die sechste Öffnung (36), die sechste zylindrische Bohrung (52) und der zweite Schlitz (38) so konfiguriert sind, dass eine sterile Injektionsnadelanordnung (66) gezielt darin positioniert werden kann;
wobei das obere Ende der sterilen Injektionsnadelanordnung (64) am oberen Ende (22) des sterilen Gehäuses (11) positioniert ist, wenn die sterile Injektionsnadelanordnung (64) in der fünften Öffnung (32), der fünften zylindrischen Bohrung (48) und dem ersten Schlitz (34) positioniert ist, und
wobei das obere Ende der sterilen Injektionsnadelanordnung (66) an dem oberen Ende (22) des sterilen Gehäuses (11) positioniert ist, wenn eine sterile Injektionsnadelanordnung (66) in der sechsten Öffnung (36), der sechsten zylindrischen Bohrung (52) und dem zweiten Schlitz (38) positioniert ist.

## Revendications

1. Support stérile (10, 10', 10") pour supporter des instruments chirurgicaux stériles d'une manière stérile disposée verticalement, comprenant :
un boîtier stérile (11) comprenant une extrémité supérieure (22) disposée horizontalement et une extrémité inférieure (12) ;
une première ouverture (24) formée dans ladite extrémité supérieure (22) dudit boîtier stérile (11) ;
une deuxième ouverture (26) formée dans ladite extrémité supérieure (22) dudit boîtier stérile (11) ;
une troisième ouverture (28) formée dans ladite extrémité supérieure (22) dudit boîtier stérile (11) ;
une quatrième ouverture (30) formée dans ladite extrémité supérieure (22) dudit boîtier stérile (11) ;
une cinquième ouverture (32) formée dans ladite extrémité supérieure (22) dudit boîtier stérile (11) ;
une première fente allongée et disposée horizontalement (34) formée dans ladite extrémité supérieure (22) dudit boîtier stérile (11), qui s'étend latéralement à partir de ladite cinquième ouverture (32) ;
une sixième ouverture (36) formée dans ladite extrémité supérieure (22) dudit boîtier stérile (11) ;
une seconde fente allongée et disposée horizontalement (38) formée dans ladite extrémité supérieure (22) dudit boîtier stérile (11) qui s'étend latéralement à partir de ladite sixième ouverture (36) ;
un premier alésage cylindrique allongé et disposé verticalement (40) formé dans ledit boîtier stérile (11) qui possède des extrémités supérieure et inférieure ;
ladite extrémité supérieure dudit premier alésage cylindrique (40) étant en communication avec ladite première ouverture (24) et s'étendant vers le bas à partir de celle-ci en direction dudit fond dudit boîtier stérile (11), grâce à quoi ladite extrémité inférieure dudit premier alésage cylindrique (40) est fermée ;
un deuxième alésage cylindrique allongé et disposé verticalement (42) formé dans ledit boîtier stérile (11) qui possède des extrémités supérieure et inférieure ;
ladite extrémité supérieure dudit deuxième alésage cylindrique (42) étant en communication avec ladite deuxième ouverture (26) et s'étendant vers le bas à partir de celle-ci en direction dudit fond dudit boîtier stérile (11), grâce à quoi ladite extrémité inférieure dudit deuxième alésage cylindrique (42) est fermée ;
ladite première ouverture (24) dans ladite extrémité supérieure (22) dudit boîtier stérile (11) et ledit premier alésage cylindrique (40) étant conçus pour comporter une première seringue stérile allongée (56), avec des extrémités supérieure et inférieure, positionnées de manière sélective en leur sein ;
ladite deuxième ouverture (26) dans ladite extrémité supérieure (22) dudit boîtier stérile (11) et ledit deuxième alésage cylindrique (42) étant conçus pour comporter une seconde seringue stérile allongée (58), avec des extrémités supérieure et inférieure, positionnées de manière sélective en leur sein ;
un troisième alésage cylindrique allongé et disposé verticalement (44) possédant des extrémités supérieure et inférieure ;
ladite extrémité supérieure dudit troisième alésage cylindrique (44) étant en communication avec ladite troisième ouverture (28) et s'étendant vers le bas à partir de celle-ci en direction dudit fond dudit boîtier stérile (11), grâce à quoi ladite extrémité inférieure dudit troisième alésage cylindrique (44) est fermée ;
ladite troisième ouverture (28) et ledit troisième alésage cylindrique (44) étant conçus pour comporter une canule à pointe arrondie allongée et stérile ou une aiguille épidurale stérile (60), possédant des extrémités supérieure et inférieure, positionnées en leur sein ;
un quatrième alésage cylindrique allongé et disposé verticalement (46) possédant des extrémités supérieure et inférieure ;
ladite extrémité supérieure dudit quatrième alésage cylindrique (46) étant en communication avec ladite quatrième ouverture (30) et s'étendant vers le bas à partir de celle-ci en direction dudit fond dudit boîtier stérile (11), grâce à quoi ladite extrémité inférieure dudit quatrième alésage cylindrique (46) est fermée ;
ladite quatrième ouverture (30) et ledit quatrième alésage cylindrique (46) étant conçus pour comporter une canule à pointe arrondie allongée et stérile ou une aiguille épidurale stérile (62), possédant des extrémités supérieure et inférieure, positionnées en leur sein ;
un cinquième alésage cylindrique allongé (48) possédant des extrémités supérieure et inférieure ;
ladite extrémité supérieure dudit cinquième alésage cylindrique (48) étant en communication avec ladite cinquième ouverture (32) et s'étendant vers le bas à partir de celle-ci en direction dudit fond dudit boîtier stérile (11), grâce à quoi ladite extrémité inférieure dudit cinquième alésage cylindrique (48) est fermée ;
ladite extrémité supérieure (22) dudit boîtier stérile (11) étant espacée au-dessus de ladite extrémité inférieure dudit boîtier stérile (11), grâce à quoi lesdites extrémités supérieures desdites première et seconde seringues allongées et stériles (56, 58) sont positionnées de manière adjacente à ladite extrémité supérieure (22) dudit boîtier stérile (11) lorsque lesdites première et seconde seringues allongées et stériles (56, 58) sont positionnées dans ladite première ouverture (24) et ledit premier alésage cylindrique (40) et ladite deuxième ouverture (26) et ledit deuxième alésage cylindrique (42), respectivement ;
ladite extrémité supérieure (22) dudit boîtier stérile (11) étant espacée au-dessus de ladite extrémité inférieure dudit boîtier stérile (11), grâce à quoi lesdites extrémités supérieures desdites canules à pointe arrondie allongées et stériles ou desdites aiguilles épidurales stériles (60, 62) sont positionnées de manière adjacente à ladite extrémité supérieure (22) dudit boîtier stérile (11) lorsque lesdites canules à pointe arrondie stériles ou lesdites aiguilles épidurales stériles (60, 62) sont positionnées dans ladite troisième ouverture (28) et ledit troisième alésage (44) et ladite quatrième ouverture (30) et ledit quatrième alésage (46), respectivement ;
ladite première fente disposée horizontalement (34) dans ledit boîtier stérile (11) possédant une extrémité supérieure et une extrémité inférieure ;
ladite cinquième ouverture (32), ledit cinquième alésage cylindrique (48) et ladite première fente (34) étant conçus pour comporter un ensemble d'aiguille d'injection stérile (64), avec des extrémités supérieure et inférieure, positionnées de manière sélective en leur sein ;
un sixième alésage cylindrique allongé (52) possédant des extrémités supérieure et inférieure ;
ladite extrémité supérieure dudit sixième alésage cylindrique (52) étant en communication avec ladite sixième ouverture (36) ;
ladite seconde fente disposée horizontalement (38) dans ledit boîtier stérile (11) possédant une extrémité supérieure et une extrémité inférieure ;
ladite sixième ouverture (36), ledit sixième alésage cylindrique (52) et ladite seconde fente (38) étant conçus pour comporter un ensemble d'aiguille d'injection stérile (66) positionné de manière sélective en leur sein ;
l'extrémité supérieure de l'ensemble d'aiguille d'injection stérile (64) étant positionnée au niveau de l'extrémité supérieure (22) dudit boîtier stérile (11) lorsque l'ensemble d'aiguille d'injection stérile (64) est positionné dans ladite cinquième ouverture (32), ledit cinquième alésage cylindrique (48) et ladite première fente (34) ; et
l'extrémité supérieure de l'ensemble d'aiguille d'injection stérile (66) étant positionnée au niveau de l'extrémité supérieure (22) dudit boîtier stérile (11) lorsqu'un ensemble d'aiguille d'injection stérile (66) est positionné dans ladite sixième ouverture (36), ledit sixième alésage cylindrique (52) et ladite seconde fente (38).
